# EUROPEAN PATENT APPLICATION

(11) **EP 0 532 119 A1**
(43) Date of publication of application: **17.03.1993**
(21) Application number: 92202744.6
(22) Date of filing: 09.09.1992
(51) Int. Cl.: A23J 3/06, A61K 7/48, A23G 3/00, A23L 1/314, A23L 1/0562, A23L 1/308

(54) **Composition comprising collagen fibre**

(30) Priority: 09.09.1991 NL 9101520
(71) Applicant: STORK FIBRON B.V., NL-5349 AE Oss (NL)
(72) Inventor: French, James William Leonard, NL-5343 JG Oss (NL)
(74) Representative: Hoijtink, Reinoud

(57) **Abstract**

The present invention relates to a composition comprising substantially fibrous collagen fibre, wherein the collagen fibre is in its substantiallly native form and preferably prepared according to PCT-application PCT/GB91/02289. The composition further comprises one or more organic or anorganic constituents, selected from the group consisting of oils, fats, water, proteins, carbohydrates or combinations thereof.

The invention further relates to the use of the compositions of the invention in or as food products, cosmetic products and therapeutical products, as well as to the preparation of the claimed compositions.

## Description

The present invention relates to a novel composition to be used as or in food products, cosmetic products and therapeutical products.

It is often desirable to control the consistency and structure of various products in view of consumer's wishes, manufacturing conditions and the like. The consistency of a product is often controlled by means of binding agents such as gelatine, collagen pulp, phosphates, alginates or other chemicals. Said binding agents are not always suited for the intended purpose because of insufficient binding properties, influencing of the taste and/or smell of the product or the like. Other problems comprise non-compatibility to the product, like certain chemicals in food products, or the occurence of unwanted side-effects, like allergic reactions to cosmetic products. Furthermore it has so far not been possible to actively control the structure of a product.

It is the object of the present invention to provide a novel composition, the consistency and structure of which can be actively controlled.

The invention therefor provides a composition comprising collagen fibre. The composition usually comprises one or more other constituents that are selected from the group consisting of water, oils and/or fats, proteins, carbohydrates.

It is known from PCT-application WO 90/10393 to reconstitute meat products by the addition of collagen fibre thereto. The starting material in said use is a complex product comprising traces of collagen. The structure and texture of the starting material are poor, but not absent, because the amount of collagen in the starting material is reduced as compared to natural meat. The method as disclosed in WO 90/10393 is used for supplementing the collagen in order to restore the natural structure of the product. The constituents of the invention are however relatively simple products with substantially no natural structure, texture or consistency. It is surprising to find that the collagen fibre also provides simple, structureless, substantially liquid or solidified liquid products, that do not comprise collagen, with a desired structure and consistency.

The particular advantage of compositions comprising collagen fibres is that the consistency and structure of the product can be controlled by the addition of only one component, namely the fibre. The fibre provides the binding function as well as the structure of the product. Furthermore it may act as an emulsifying agent for the various constituents of the composition.

The collagen fibre to be used is preferably prepared according to PCT Patent Application PCT/GB91/02289. The method disclosed therein provides collagen in which the fibrous character of the collagen is retained. The fibre has better binding properties, a higher cook out temperature and does not comprise granula. The fibre used in the invention is preferably not oxidized and/or hydrolyzed and has a very low water content, preferably less than 7%, more preferably less than 5%. The fibre has a feather-like appearance. Said feather structure provides a bridge between the other constituents of the composition, thus acting as a binder. The fibres also provide the product with the desired structure.

It is also possible to use collagen fibres that are prepared otherwise as long as the collagen is in a fibrous and substantially native form although the collagen fibre prepared according to PCT/GB91/02289 is preferred.

As already mentioned above it is possible to mix the fibre with various other constituents, each of them resulting in a different product but having as a common feature that the consistency and structure thereof are controlled by means of the collagen fibre.

In case said other constituent is an oil the consistency of the product is a creamy one. By using oils like lanolin, olein etc. creams are prepared that may form a diluent or excipient for a cosmetic product. It has been found that the addition of the fibre to the oil reduces the greasy character thereof. The addition of fragrances may produce a hand, facial or bodycream. The use of the collagen fibre leaves behind a thin collagen film on the skin after evaporation of the other constituents leaving the skin smooth and well protected against dehydration and other harmfull effects. The collagen film formed on the skin is also waterresistent.

To produce a product with a lower fat content part of the oil or fat may be replaced by water. The collagen fibre replaces the binding agents formerly used in cosmetic creams, makes it easily spreadable and provides the cream with a pleasant texture. Since collagen is a natural constituent of the skin the product obtained with the composition is likely to be hypo-allergenic, since the use of chemical binding agents may be omitted. The use of collagen in creams is widely spread. The collagen of the invention however is collagen in a fibrous form. The use of said form of collagen in cosmetic products is still novel.

In combination with an antiseptic or other therapeutical substances the composition could be used as a diluent for the preparation of a therapeutical cream that is useful for desinfecting or treating the skin, while protecting it by means of the collagen film. The protecting function of the fibre is in particular useful for preventing cracked hands or other skin damages caused by e.g. working with aggresive or dehydrating substances or for healing wounds, burns or cuts.

Mixing the collagen fibre into a mixture of water and oil or fat will produce a stable oil-in-water or water-in-oil emulsion depending on the amounts of water and oil or fat used. Thus the fibre serves as an emulsifying agent.

Other constituent that may be added to the composition comprise proteins and carbohydrates. Said proteins may be selected from the group consisting of soya isolate, sodium caseinate, etc. The carbohydrates may be selected from carbohydrates originating from plants, such as various corns, rice, beets, potatoes.

The combination of proteins, water and collagen fibre provides a tempeh- or tofu-like product to be used as a meat substitute for e.g. a vegetarian diet. Due to the addition of the fibre the product is provided with a so-called "bite". The protein to be used could be soya isolate or any other nutritious protein or protein product, particularly originating from vegetables and vegetation. Other options comprise milk proteins, like sodium caseinate, vegetable flours, such as nut flour etc.

In case water, collagen fibre and carbohydrate are mixed a product is obtained that might be used as a confectionary product, such as wine gums, candy bars, quickly setting desserts etc.. In said products at least part of the gelatine may be replaced by the fibre that also provides the product with a certain structure.

Natural untreated fats like cutting fat, trim fat together with water, collagen fibre and a protein powder result in an emulsion that can be used in meat products like luncheon meat, ham etc. to replace the more expensive and less stable animal fat normally used therein. The above constituents are preferably used in a ratio of 5:5:0.5:0.5. The emulsion of the invention will be stable upon cooking, grilling, sterilisation, pasteurisation etc. and not cook out of the product to a temperature of up to 135°C.

In case vegetable oils are used to replace the fat in the above composition the product thus obtained is in particular useful for so-called "light products" since they are low in cholesterol because of the reduced animal fat content. Equal amounts of vegetable oil, water, fibre and protein will result in a margarine like product.

Both the above described fat and oil based compositions can also be used in petfoods.

Rendered, i.e. melt out, fat or lard with water, collagen fibre and protein powder produces an excellent sliceable product that can be used as backfat for coating meatproducts like Berliner liverwurst and pâté. Backfat was previously obtained from pigs. Present pigs however become more and more lean and do therefore not produce enough backfat to meet the needs of the market. A substitute fat is therefore highly desirable. The artificial backfat of the invention is therefore of particular economic importance.

The backfat of the emulsion preferably comprises 4 to 6 parts of an animal fat, 6 to 4 parts of water, at least one half part of a protein product and at least on half part of the collagen fibre.

The amounts and properties of the constituents that are mixed determine the consistency of the obtained products. Oil will give a more cream-like product, whereas fat will yield a paste-like composition. The amount of fibre used will determine both the consistency and the structure of the product. In general it can be said that the consistency of the product can vary between a liquid and a solid.

It is also possible to swell the fibre in an acid or alkaline solution before adding it to the composition. The fibrous structure is then partially lost. In certain applications such a partially denatured fibre may be usefull.

In addition to the already described additives that determine the use of the composition of the invention other additives might be added, such as preservatives, like sulfite, anti-oxydants, like ascorbate, stabilising agents, emulsifying agents, flavors, fragrances, colouring agents and the like. For use in food products food compatible additives are to be used as will be readily clear to someone skilled in the art. In case of cosmetic and therapeutical products cosmetically and therapeutically acceptable excipients are to be used.

The present invention will be exemplified by the following examples which are only given by way of illustration and are in no way intended to limit the scope of the present invention. In the examples the term part stands for part by weight unless otherwise indicated.

### EXAMPLE 1

### Preparation of a basis for a cosmetic or therapeutical cream.

Equal amounts of lanolin, water and collagen fibre are mixed under high shear to obtain a basis for a cosmetic or therapeutical cream. The addition of fragrances, an antispetic, and/or other additives yields the final product. The cream thus obtained is smooth and soft, has a pleasant feel, is easily spreadable and leaves the skin smooth and well protected.

### EXAMPLE 2

### Preparation of a fat substitute for use in meat products

Composition :
10 parts of trim fat, cutting fat
10 parts of water,
1 part of soya isolate, and
1 part of collagen fibre.

The fat is chopped in a bowlcutter at 5°C. Then the protein powder and water are added and the thus obtained mixture is again chopped in the bowlcutter at 5°C. After adding the collagen the mixture is once more chopped in the bowlcutter at approximately 10°C. The thus obtained product can be used as a fat substitute in e.g. meat products.

### EXAMPLE 3

### Preparation of an artificial backfat

Composition :
10 parts rendered fat or lard
10 parts water
1 part soya isolate or sodium caseinate
1 part collagen fibre
A product is prepared according to example 2. The product may be poured into a form and left to set to obtain a block of which backfat layers may be sliced. It is also possible to extrude the product in order to obtain a backfat layer.

### EXAMPLE 4

### Preparation of a meat substitute

Composition :
5 parts of water
5 parts of protein such as soya isolate,
1 part of collagen fibre
The ingredients are mixed to obtain a meat substitute.

### EXAMPLE 5

### Preparation of a confectionary product

Composition A:
7 parts of water
5 parts of carbohydrates, such as sugars, starches etc.
1 part of collagen fibre

Composition B:
7 parts of water
5 parts of carbohydrates, such as sugars, starches etc.
1 part of collagen fibre
0.25 part of arabic gum

Composition C:
7 parts of water
5 parts of carbohydrates, such as sugars, starches etc.
1 part of collagen fibre
0.5 part of a polyalcohol, such as glycerol, sorbitol etc.
The ingredients are mixed to obtain a confectionary product. Product A is a rather hard product, whereas the products B and C are softer due to the addition of plasticizing agents.

## Claims

1. Composition comprising substantially fibrous collagen fibre.

2. Composition as claimed in claim 1, **characterized in that** the collagen fibre is in its substantiallly native form.

3. Composition as claimed in claim 1 or 2, **characterized in that** the collagen fibre is a fibre prepared according to PCT-application PCT/GB91/02289.

4. Composition as claimed in claim 1, 2 or 3, **characterized in that** the composition further comprises one or more organic or anorganic constituents.

5. Composition as claimed in claim 4, **characterized in that** the constituent is an oil or fat, or combinations thereof.

6. Composition as claimed in claim 5, **characterized in that** the oil or fat is a vegetable-based oil or fat.

7. Composition as claimed in claim 6, **characterized in that** the vegetable-based oil or fat is an oil or fat selected from the group consisting of coconut oil, sunflower oil, or combinations thereof.

8. Composition as claimed in claim 5, **characterized in that** the oil or fat is an animal oil or fat.

9. Composition as claimed in claim 8, **characterized in that** the animal oil or fat is an oil or fat selected from the group consisting of lard, cutting fat, trim fat, lanolin, olein, or combinations thereof.

10. Composition as claimed in any one of the claims 5-9, **characterized in that** the constituent is a combination of one or more vegetable-based and one or more animal fats and/or oils.

11. Composition as claimed in any one of the claims 5-10, **characterized in that** the composition further comprises water.

12. Composition as claimed in claim 11, **characterized in that** the composition further comprises one or more proteins.

13. Composition as claimed in claim 12, **characterized in that** the protein is selected from a group consisting of soya isolate, sodium caseinate.

14. Composition as claimed in claim 4, **characterized in that** the constituent is water and the composition further comprises one or more carbohydrates.

15. Composition as claimed in claim 14, **characterized in that** the carbohydrates are originating from plants such as various corns, rice, beets, potatoes, or combinations thereof.

16. Composition as claimed in claim 4, **characterized in that** the constituent is water and the composition further comprises one or more proteins.

17. Composition as claimed in claim 16, **characterized in that** the proteins are selected from the group consisting of soya isolate, sodium caseinate, or combinations thereof.

18. Method for preparing a composition as claimed in any one of the claims 1-17.

19. Use of the composition as claimed in any one of the claims 1-17 as a diluent for cosmetic and/or therapeutic products.

20. Use of the composition as claimed in any one of the claims 1-17 for the preparation of an edible product.
